# EUROPEAN PATENT SPECIFICATION

(11) **EP 2 707 018 B1**
(45) Date of publication and mention of the grant of the patent: **04.11.2015**
(21) Application number: 12719752.3
(22) Date of filing: 10.05.2012
(51) Int. Cl.: A61K 38/43, A61K 38/51, A61P 9/02

(54) **METHODS AND PHARMACEUTICAL COMPOSITIONS FOR THE TREATMENT OF AUTOIMMUNE DISEASES**
VERFAHREN UND PHARMAZEUTISCHE ZUSAMMENSETZUNGEN ZUR BEHANDLUNG VON AUTOIMMUNERKRANKUNGEN
PROCÉDÉS ET COMPOSITIONS PHARMACEUTIQUES POUR LE TRAITEMENT DE MALADIES AUTO-IMMUNES

(30) Priority: 10.05.2011 EP 11305557
(43) Date of publication of application: 19.03.2014
(73) Proprietor: Institut National de la Santé et de la Recherche Médicale (INSERM), 75013 Paris (FR); Universite de Rouen, 76130 Mont-Saint-Aignan (FR); Centre Hospitalier Universitaire de Rouen, 76000 Rouen (FR)
(72) Inventor: VITTECOQ, Olivier, F-76183 ROUEN Cedex (FR); DERAMBURE, Céline, F-76183 ROUEN Cedex (FR); LEQUERRE, Thierry, F-76183 ROUEN Cedex (FR); BOYER, Olivier, F-76183 ROUEN Cedex (FR); TRON, François, F-76183 ROUEN Cedex (FR); LE LOET, Xavier, F-76183 ROUEN Cedex (FR); GILBERT, Danièle, F-76183 ROUEN Cedex (FR)
(74) Representative: Cabinet Plasseraud
(86) International application number: PCT/EP2012/058678
(87) International publication number: WO 2012/152882

(56) References cited:
- WO-A1-2008/090360
- WO-A1-2012/138294
- WO-A2-2010/117694
- ARC: "Prophylactic injection of non-citrullinated aplha-enolase has immonomodulatory effects in callagen-induced arthritis mice.", , 2011, XP002660174, Retrieved from the Internet: URL:http://acr.confex.com/acr/2011/webprog ram/Paper22153.html [retrieved on 2011-09-28]
- WEGNER NATALIA ET AL: "Autoimmunity to specific citrullinated proteins gives the first clues to the etiology of rheumatoid arthritis.", IMMUNOLOGICAL REVIEWS JAN 2010 LNKD- PUBMED:20192991, vol. 233, no. 1, January 2010 (2010-01), pages 34-54, XP002660175, ISSN: 1600-065X
- TERRIER ET AL: "Alpha-enolase: A target of antibodies in infectious and autoimmune diseases", AUTOIMMUNITY REVIEWS, ELSEVIER, AMSTERDAM, NL, vol. 6, no. 3, 5 February 2007 (2007-02-05), pages 176-182, XP005876579, ISSN: 1568-9972, DOI: 10.1016/J.AUTREV.2006.10.004
- KINLOCH A J ET AL: "PATHOGENIC ROLE OF ANTIBODIES TO CITRULLINATED PROTEINS IN RHEUMATOID ARTHRITIS", EXPERT REVIEW OF CLINICAL IMMUNOLOGY, FUTURE DRUGS LTD., LONDON, GB, vol. 2, no. 3, 1 May 2006 (2006-05-01), pages 365-375, XP001248199, ISSN: 1744-666X, DOI: 10.1586/1744666X.2.3.365
- LUNDBERG KARIN ET AL: "Antibodies to citrullinated alpha-enolase peptide 1 are specific for rheumatoid arthritis and cross-react with bacterial enolase.", ARTHRITIS AND RHEUMATISM OCT 2008 LNKD- PUBMED:18821669, vol. 58, no. 10, October 2008 (2008-10), pages 3009-3019, XP002660176, ISSN: 0004-3591

## Description

### FIELD OF THE INVENTION:

The present invention relates to methods and pharmaceutical compositions for the treatment of autoimmune diseases, especially rheumatoid arthritis.

### BACKGROUND OF THE INVENTION:

Rheumatoid arthritis (RA), whose prevalence is around 1% in the world, is a chronic inflammatory disorder characterized by synovial hyperplasia leading to joint destruction. This disease affects more often 35-50 years-old women with a sex-ratio=4/l and is responsible of major disability. In this regard, it represents a public health problem.

The aetiology of RA is still unknown. Several causes have been proposed including: 1) environmental factors (tobacco, microbial pathogens), 2) genetic factors (susceptibility alleles HLA-DRB1*0401 and *0404 in caucasian population, allele T 1858 of PTPN22), 3) hormonal factors 4) immunological factors. Both innate and adaptative immunity are involved in the onset and the maintenance of the disease respectively. Many cells (Antigen presenting cells, T cells, T regulatory cells and B cells) are involved in the pathogenesis of RA. This multicellular system plays a pivotal role in the different phases of the disease with direct cell interactions and cytokinic communication (TNF-alpha, IL-1beta, IL-1 Ra, IL-4, IL-10, IL-17, RANK-L). Although the different factors mentioned above interact for the development of RA, it is likely that autoimmune factors play a central role in RA pathogenesis.

Identification of autoantibodies in RA patients has been of major interest and the search is still actively ongoing. To date, autoantibodies recognizing citrullinated self-antigens have been reported to be the most specific (98%) autoantibodies in RA. Peptidyl-citrulline residues in proteins are produced only through a post-translational modification of arginines catalyzed by peptidyl-arginine deiminase (PAD) with a subsequent change in antigenicity of the self-proteins. This reaction, called citrullination, is involved in multiple inflammatory processes, but the anti-citrullinated peptides autoantibodies (ACPA) are highly specific of RA. In fact, ACPAs cover a vast array of antigenic specificities, including antigenic determinants present in several proteins

In this context, a previous analysis of new populations of autoantibody in very early RA by a proteomic approach (2D electrophoresis coupled to MALDI-TOF mass spectrometer), allowed to identify for the first time α-enolase as a new auto-antigen from 255 very early RA patients. Further studies, performed, highlighted two categories of auto-antigens targeted by the early untreated RA autoantibody response with some of them citrullinated: 1) enzymes of the glycolytic family and notably citrullinated α-enolase (ENO) and 2) molecular chaperones including BIP, another well documented target antigen in RA. Another group has shown that citrullination of ENO is crucial for its autoantigenicity compared to the non-citrullinated form. In this respect, 46% of tested RA sera showed reactivity against the citrullinated form and only 13% against the non-citrullinated form.

alpha-Enolase is a multifunctional protein expressed in various tissues such as liver, thymus, kidney and also in synovial tissue. It is a key glycolytic enzyme which converts 2-phospho-glycerate into phosphoenolpyruvate. In addition to this property, alpha-Enolase exerts several biological functions related to its cellular localization. Its ability to serve as a plasminogen receptor on the surface of a variety of cells (epithelial, endothelial and hematopoietic cells) suggests that it can play a role in the intravascular and pericellular fibrinolysis. When neutrophils, monocytes, B and T cells are stimulated by phorbolmyristateacetate and LPS, membrane expression of alpha-Enolase is up-regulated. Myc binding protein 1 (MBP-1), an alternative translation initiation product of the alpha-Enolase RNA, may be found in the nucleus, where it acts as a transcriptional repressor of the c-myc proto-oncogene, with subsequent regulation of cell growth and differentiation.

ENO is a target of antibodies in a wide spectrum of infectious and autoimmune diseases ENO such as ulcerative colitis, Crohn's disease, primary sclerosing cholangitis, systemic lupus erythematosus, autoimmune hepatitis, Behçet's disease. Antibodies against ENO have been found in 0 to 6% of healthy controls. Nevertheless, to date, the presence of autoantibodies against citrullinated ENO remains exclusive of RA. It has been proposed that the pathogenic role of ENO antibodies may be explained by a molecular mimicry process. An ENO immunodominant peptide was identified and referred as to EP 1. Antibodies to this epitope were observed in 37-62% of sera obtained from patients with RA. This immunodominant peptide showed 82% homology with a peptide of ENO from *Porphyromonas gingivali* (referred as to por-EP1). The levels of antibodies to this immunodominant peptide correlated highly with the levels of antibodies to the bacterial peptide. Moreover, antibodies to the human peptide cross-react with citrullinated recombinant *Porphyromonas gingivalis* enolase. These data may indicate a role for bacterial infection in priming autoimmunity in a subset of patients with RA

However arthritogenic or immunomodulatory properties of ENO has not yet been investigated.

### SUMMARY OF THE INVENTION:

The present invention relates to an α-enolase polypeptide for use in the prophylactic treatment of an autoimmune disease in a subject in need thereof as defined by the claims.

### DETAILED DESCRIPTION OF THE INVENTION:

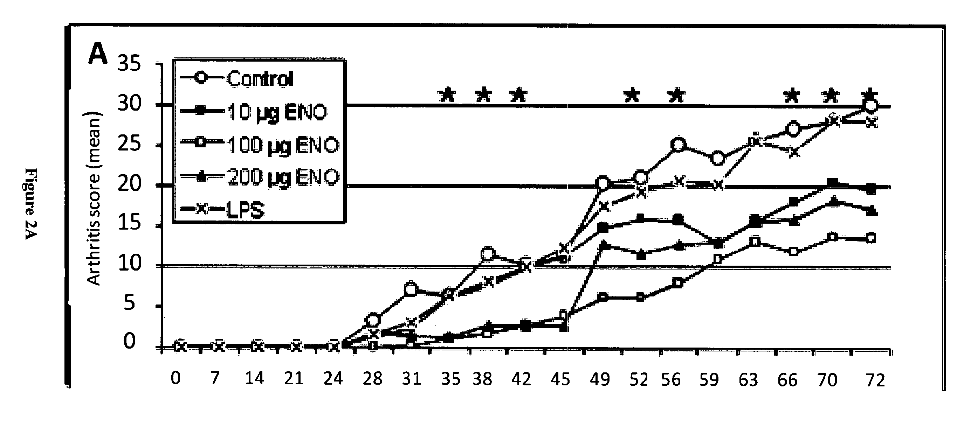

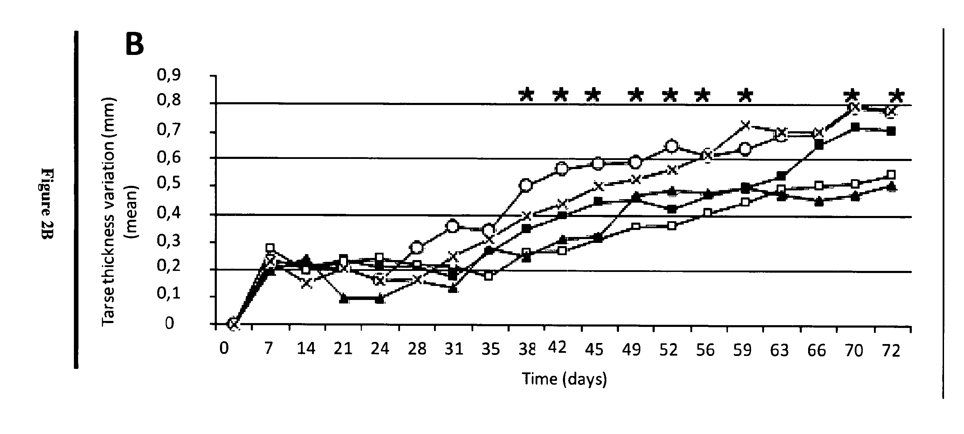

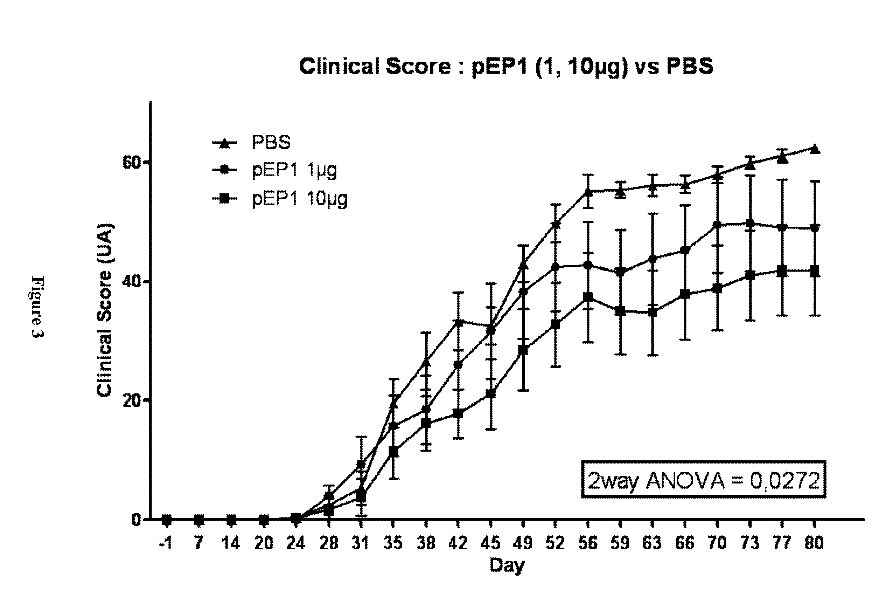

The inventors have evaluated the clinical and immunological effects of recombinant non-citrullinated α-enolase in the well-known collagen induced athritis model. Different doses (10 - 100 µg) of α-enolase were indeed intraperitoneally injected to 6 week-old DBA/1 mice one day prior to collagen II arthritis induction. Both clinical (weight, global and joint scores, tarsal thickness) and biological (anticollagen II and anti-α-enolase [home-made ELISA] antibodies, cytokines [IL-1b, IL-2, TNF-a, IL-6, IL-4, IL-10, IL-17, IFNg]) levels were assessed during a 72 days follow-up period. Across 2 different experiments, prophylactic injection of recombinant α-enolase was able to prevent weight loss and to decrease the severity of arthritis evaluated by the global and joint scores as well as the tarsal thickness. There was a dose-effect since 100 µg led to better results (Figure 2). In the same way, prophylactic injection of por-EP1 led to the same observations with, once again, a dose effect (Figure 3)

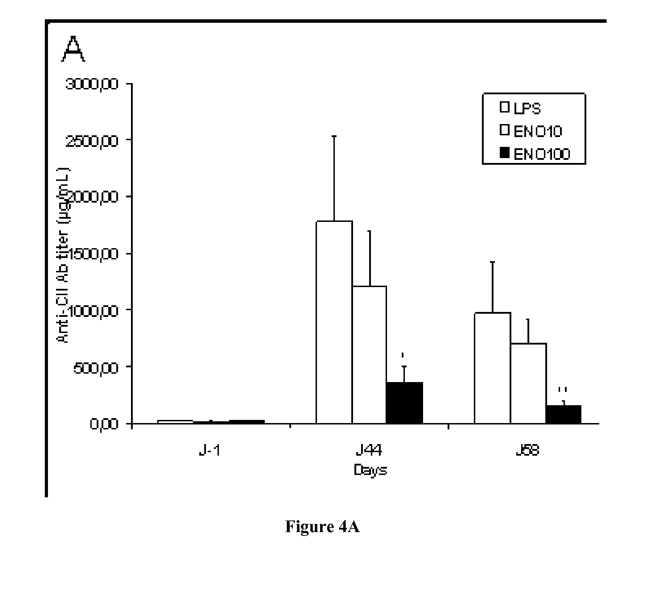

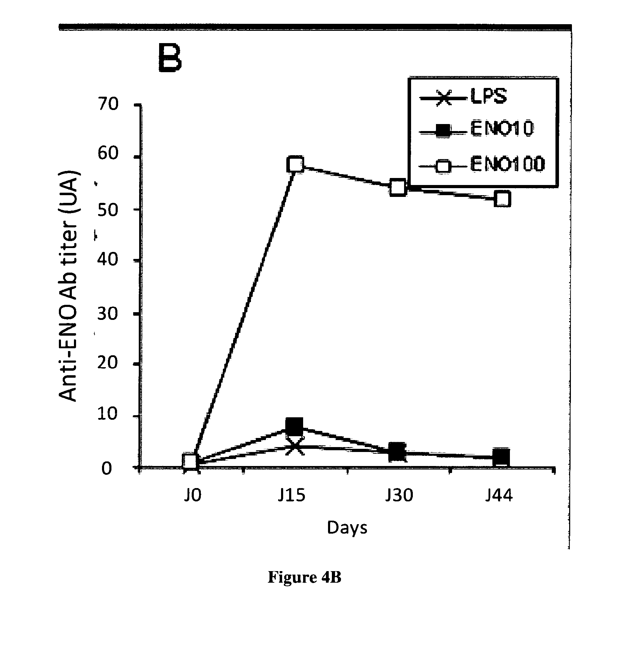

The immunomodulatory effects of both recombinant ENO and the immunodominant peptide por-EP1 being observed, several experiments were performed to understand the mechanisms induced by this molecule. Levels of anti-collagen II antibodies were significantly lower whereas titers of anti-α-enolase antibodies were significantly higher in mice treated with 100 µg of α-enolase compared to control mice (Figure 4). By Luminex technology, an experiment suggest that pro-inflammatory cytokines are decreased from day 58 in 100 µg treated mice compared to control mice. Furthermore, in-vitro studies showed that recombinant ENO incubated with PBMCs from healthy blood donors induced the production of IL-10. Taken together, those cytokine experiments suggest the induction of an immune deviation (TH2 profile) by recombinant ENO injected in a prophylactic way. In conclusion, prophylactic treatment with recombinant α-enolase or por-EP1 has immunomodulatory effects in collagen induced arthritis mice. The regulatory mechanisms induced by this protein seem to be partially due to a control of the production of anti-collagen II antibodies and of the Th1 response. Those results suggest that non-citrullinated alphα-enolase polypeptides or fragments thereof (including function conservative variants thereof such as por-EP1) represent a new therapeutic approach in RA.

Accordingly, the present invention relates to an α-enolase polypeptide for use in the prophylactic treatment of an autoimmune disease in a subject in need thereof.

The prophylactic administration of α-enolase polypeptide of the invention should serve to prevent or attenuate said autoimmune disease in said subject. In a preferred embodiment subject, preferably human, at high risk for an autoimmune disease are prophylactically treated with the α-enolase polypeptide of the invention. Examples of such subjects include, but are not limited to, humans with a family history of autoimmune disease. In a preferred embodiment, said autoimmune disease result in part of the presence of antoantibodies against citrullinated α-enolase. In another particular preferred embodiment, said automimmne disease is rheumatoid arthritis.

The term "α-enolase" or "ENO" has its general meaning in the art and refers to the glycolytic enzyme which converts 2-phospho-glycerate into phosphoenolpyruvate. The term includes naturally occurring α-enolase and function conservative variants and modified forms thereof.

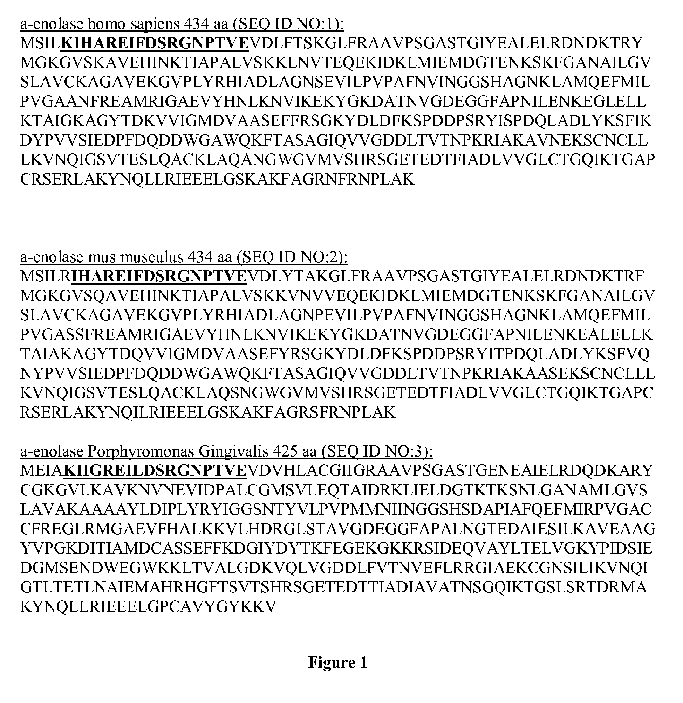

The α-enolase can be from any source, but typically is a mammalian (e.g., human and non-human primate) α-enolase, and more particularly a human α-enolase. The sequence of α-enolase protein and nucleic acids for encoding such a protein are well known to those of skill in the art. An exemplary sequence of human α-enolase is provide as set forth in SEQ ID NO:1 and an exemplary sequence of murine α-enolase is provide as set forth in SEQ ID NO:2 [Figure 1]. However, it should be understood that, as those of skill in the art are aware of the sequence of these molecules, any α-enolase protein or gene sequence variant may be used as long as it has the properties of an α-enolase.

"Function conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" is a polypeptide which has at least 60 % amino acid identity as determined by BLAST or FASTA algorithms, preferably at least 75 %, most preferably at least 85%, and even more preferably at least 90 %, and which has the same or substantially similar properties or functions as the native or parent protein to which it is compared.

According to the invention the term "α-enolase polypeptide" refers to any polypeptide that comprises the immunodominant fragment of α-enolase EP1 (or function conservative variant thereof) consisting of the amino acid sequence ranging from position 5 to position 21 in SEQ ID NO:1 or ranging from position 6 to position 21 in SEQ ID NO:2 (Figure 1). Accordingly, the term encompasses α-enolase itself or fragments thereof comprising the immunodominant fragment of α-enolase EP1.

In a particular embodiment, a function conservative variant of the immunodominant fragment EP1 may be represented by the peptide ranging from position 5 to position 21 in SEQ ID NO:3 (por-EP1).

According to the invention all polypeptides of the invention are recombinant polypeptides that are not citrullinated.

In a particular embodiment, α-enolase polypeptides of the invention can be conformationally constrained to retain their immunogenicity properties.

Typically, cyclization is well known in the art and generally involves the introduction of a disulfide bound between two cysteine residues. Typically, the cycle is formed through a side chain to side chain ring involving a monosulfide or disulfide bridge between pairs of cysteines, penicillamines, homocysteines, combinations of the foregoing, or other pairs of amino acids in which the side chains are linked with either one or two sulfur atoms. Methods for the synthesis of disulfide cyclic polypeptide are well known in the art and are described for example in US3,929,758, US4,216,141; and US4,102,877.

Polypeptides of the invention may thus comprise cysteine residues at terminal ends to allow the cyclisation of the polypeptides.

In a particular embodiment, the α-enolase polypeptide is selected from the group consisting of CKIHAREIFDSRGNPTVEC (SEQ ID NO:4), CIHAREIFDSRGNPTVEC (SEQ ID NO:5) or CKIIGREILDSRGNPTVEC (SEQ ID NO:6) that are cyclised by a disulfide bound between the two cysteine residues.

In specific embodiments, it is contemplated that α-enolase polypeptides used in the therapeutic methods of the present invention may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG), has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomerular filtration (e.g., less than 45 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes (see e.g., technologies of established by VectraMed, Plainsboro, N.J.). Such linkers may be used in modifying the α-enolase polypeptides described herein for therapeutic delivery.

According to the invention, α-enolase polypeptides may be produced by conventional automated peptide synthesis methods or by recombinant expression. General principles for designing and making proteins are well known to those of skill in the art.

α-enolase polypeptides of the invention may be synthesized in solution or on a solid support in accordance with conventional techniques. Various automatic synthesizers are commercially available and can be used in accordance with known protocols. α-enolase polypeptides of the invention may also be synthesized by solid-phase technology employing an exemplary peptide synthesizer such as a Model 433A from Applied Biosystems Inc. The purity of any given protein; generated through automated peptide synthesis or through recombinant methods may be determined using reverse phase HPLC analysis. Chemical authenticity of each peptide may be established by any method well known to those of skill in the art.

As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

A variety of expression vector/host systems may be utilized to contain and express the peptide or protein coding sequence. These include but are not limited to microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti or pBR322 plasmid); or animal cell systems. Those of skill in the art are aware of various techniques for optimizing mammalian expression of proteins. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells. Exemplary protocols for the recombinant expression of the peptide substrates or fusion polypeptides in bacteria, yeast and other invertebrates are known to those of skill in the art and a briefly described herein below. Mammalian host systems for the expression of recombinant proteins also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing which cleaves a "prepro" form of the protein may also be important for correct insertion, folding and/or function. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

In the recombinant production of the α-enolase polypeptides of the invention, it would be necessary to employ vectors comprising polynucleotide molecules for encoding the α-enolase-derived proteins. Methods of preparing such vectors as well as producing host cells transformed with such vectors are well known to those skilled in the art. The polynucleotide molecules used in such an endeavor may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding sites, and appropriate sequences which control transcription and translation.

The terms "expression vector," "expression construct" or "expression cassette" are used interchangeably throughout this specification and are meant to include any type of genetic construct containing a nucleic acid coding for a gene product in which part or all of the nucleic acid encoding sequence is capable of being transcribed.

The choice of a suitable expression vector for expression of the peptides or polypeptides of the invention will of course depend upon the specific host cell to be used, and is within the skill of the ordinary artisan. Methods for the construction of mammalian expression vectors are disclosed, for example, in EP-A-0367566; and WO 91/18982.

In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are well known in the art.

Preferred viruses for certain applications are the adenoviruses and adeno-associated (AAV) viruses, which are double-stranded DNA viruses that have already been approved for human use in gene therapy. Actually 12 different AAV serotypes (AAV1 to 12) are known, each with different tissue tropisms. Recombinant AAV are derived from the dependent parvovirus AAV2. The adeno-associated virus type 1 to 12 can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Expression requires that appropriate signals be provided in the vectors, such as enhancers/promoters from both viral and mammalian sources that may be used to drive expression of the nucleic acids of interest in host cells. Usually, the nucleic acid being expressed is under transcriptional control of a promoter. A "promoter" refers to a DNA sequence recognized by the synthetic machinery of the cell, or introduced synthetic machinery, required to initiate the specific transcription of a gene. Nucleotide sequences are operably linked when the regulatory sequence functionally relates to the DNA encoding the protein of interest (i.e., α-enolase, a variant and the like). Thus, a promoter nucleotide sequence is operably linked to a given DNA sequence if the promoter nucleotide sequence directs the transcription of the sequence.

Similarly, the phrase "under transcriptional control" means that the promoter is in the correct location and orientation in relation to the nucleic acid to control RNA polymerase initiation and expression of the gene. Any promoter that will drive the expression of the nucleic acid may be used. The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell. Generally speaking, such a promoter might include either a human or viral promoter. Common promoters include, e.g., the human cytomegalovirus (CMV) immediate early gene promoter, the SV40 early promoter, the Rous sarcoma virus long terminal repeat, [beta]-actin, rat insulin promoter, the phosphoglycerol kinase promoter and glyceraldehyde-3-phosphate dehydrogenase promoter, all of which are promoters well known and readily available to those of skill in the art, can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient to produce a recoverable yield of protein of interest. By employing a promoter with well known properties, the level and pattern of expression of the protein of interest following transfection or transformation can be optimized. Inducible promoters also may be used.

Another regulatory element that is used in protein expression is an enhancer. These are genetic elements that increase transcription from a promoter located at a distant position on the same molecule of DNA. Where an expression construct employs a cDNA insert, one will typically desire to include a polyadenylation signal sequence to effect proper polyadenylation of the gene transcript. Any polyadenylation signal sequence recognized by cells of the selected transgenic animal species is suitable for the practice of the invention, such as human or bovine growth hormone and SV40 polyadenylation signals.

Another aspect of the invention relates to a nucleic acid molecule encoding for an α-enolase polypeptide according to the invention for use in the prophylactic treatment of an autoimmune disease as defined above.

Typically, said nucleic acid is a DNA or RNA molecule, which may be included in any suitable vector, such as a plasmid, cosmid, episome, artificial chromosome, phage or a viral vector as above described.

So, a further object of the invention relates to a vector comprising a nucleic acid encoding for an α-enolase polypeptide for use in the prophylactic treatment of an autoimmune disease as defined above.

A further object of the invention relates to a host cell comprising a nucleic acid encoding for an α-enolase polypeptide (or a vector comprising a nucleic acid thereof) for the prophylactic treatment of an autoimmune disease as defined above.

The α-enolase polypeptides (or nucleic acid encoding for a α-enolase polypeptide or a vector comprising a nucleic acid encoding for a α-enolase polypeptide) of the invention are administered in a therapeutically effect amount in said subject.

By a "therapeutically effective amount" is meant a sufficient amount of α-enolase polypeptides (or nucleic acid encoding for a α-enolase polypeptide or a vector comprising a nucleic acid encoding for a α-enolase polypeptide) to preserve the vascular endothelial cell barrier integrity at a reasonable benefit/risk ratio applicable to any medical treatment. It will be understood that the total usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved.

A further object of the invention relates to pharmaceutical compositions comprising a α-enolase polypeptide (or nucleic acid encoding for a α-enolase polypeptide or a vector comprising a nucleic acid encoding for a α-enolase polypeptide) for use in the prophylactic treatment of an autoimmune disease.

Typically, the α-enolase polypeptide (or nucleic acid encoding for a α-enolase polypeptide or a vector comprising a nucleic acid encoding for a α-enolase polypeptide) may be combined with pharmaceutically acceptable excipients, and optionally sustained-release matrices, such as biodegradable polymers, to form therapeutic compositions.

"Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The α-enolase polypeptide (or nucleic acid encoding for a α-enolase polypeptide or a vector comprising a nucleic acid encoding for a α-enolase polypeptide) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the patient being treated. The person responsible for administration will, in any event, determine the appropriate dose for the individual patient.

In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

The invention will be further illustrated by the following figures and examples. However, these examples and figures should not be interpreted in any way as limiting the scope of the present invention.

### FIGURES:

**Figure 1****. Aminoacid sequence of native alpha-enolase from human, mouse and *Porphyromonas Gingivalis***
**Figure 2****: Clinical effects of prophylactic a-enolase injection in murine collagen-induced arthritis.**
**Figure 3****: Clinical effects of prophylactic por-EP1 injection in murine collagen-induced arthritis: Prophylactic injection (IP) of cyclic P. Gingivalis peptide (ckiigreildsrgnptvec), derivated from enolase, allows to decrease significantly arthritis severity in CIA. We can observe a dose-effect, better results were obtained with a dose of 10µg of peptide (means+/-SEM are indicated).**
**Figure 4****: prophylactic enolase injection induces both anticollagen II antibodies titer decrease and anti-enolase antibodies titer increase in murine collagen induced arthritis: (A) anti-CII Ab and (B) anti-ENO Ab titers.**

### EXAMPLES:

CIA is a well-established experimental animal model close to human RA, with which it shares many clinical, immunological and histopathological features (Courtenay, J.S., et al., Immunisation against heterologous typo II collagen induces arthritis in mice. Nature, 1980. 283(5748): p. 666-8.]. CIA can be induced in genetically (H-2^{q} or H-2^{r}) susceptible strains (DBA/1, B10.Q, B10.RIII) of mice by immunization with native heterologous collagen II (CII), a known component of cartilage. Both humoral and cellular immunity are implicated in this model, because anti-CII antibodies and CII-specific Th1 cells are necessary for the development of arthritis. T cell deficient mice do not develop disease. The resulting disease is a chronic proliferative synovitis with important cartilage destruction, bone erosion leading to joint deformations. CIA is an appropriate model, which has been widely used to evaluate approved RA therapies (etanercept, anakinra, abatacept, toclizumab) and compounds that were discontinued during phase II or III clinical trials.

Accordingly, the inventors have evaluated the clinical and immunological effects of recombinant non-citrullinated α-enolase in the well-known collagen induced athritis model. Different doses (10 - 100 µg) of α-enolase were indeed intraperitoneally injected to 6 week-old DBA/1 mice one day prior to collagen II arthritis induction. Both clinical (weight, global and joint scores, tarsal thickness) and biological (anticollagen II and anti-α-enolase [home-made ELISA] antibodies, cytokines [IL-1b, IL-2, TNF-a, IL-6, IL-4, IL-10, IL-17, IFNg]) levels were assessed during a 72 days follow-up period. Across 2 different experiments, prophylactic injection of recombinant α-enolase was able to prevent weight loss and to decrease the severity of arthritis evaluated by the global and joint scores as well as the tarsal thickness. There was a dose-effect since 100 µg led to better results (Figure 2). In the same way, prophylactic injection of por-EP1 led to the same observations with, once again, a dose effect (Figure 3)

The immunomodulatory effects of both recombinant ENO and the immunodominant peptide por-EP1 being observed, several experiments were performed to understand the mechanisms induced by this molecule. Levels of anti-collagen II antibodies were significantly lower whereas titers of anti-α-enolase antibodies were significantly higher in mice treated with 100 µg of α-enolase compared to control mice (Figure 4). By Luminex technology, an experiment suggest that pro-inflammatory cytokines are decreased from day 58 in 100 µg treated mice compared to control mice. Furthermore, in-vitro studies showed that recombinant ENO incubated with PBMCs from healthy blood donors induced the production of IL-10. Taken together, those cytokine experiments suggest the induction of an immune deviation (TH2 profile) by recombinant ENO injected in a prophylactic way. In conclusion, prophylactic treatment with recombinant α-enolase or por-EP1 has immunomodulatory effects in collagen induced arthritis mice. The regulatory mechanisms induced by this protein seem to be partially due to a control of the production of anti-collagen II antibodies and of the Th1 response. Those results suggest that non-citrullinated alphα-enolase or por-EP1 represents a potential new therapeutic approach in RA.

### REFERENCES:

Throughout this application, various references describe the state of the art to which this invention pertains.

## Claims

1. A non citrullinated α-enolase polypeptide for use in a method for the prophylactic treatment of an autoimmune disease in a subject in need thereof wherein said non citrullinated α-enolase polypeptide comprises:
an amino acid sequence selected from the group consisting of the amino acid sequence ranging from position 5 to position 21 in SEQ ID NO:1, the amino acid sequence ranging from position 6 to position 21 in SEQ ID NO:2 and the amino acid sequence ranging from position 5 to position 21 in SEQ ID NO:3; or
an amino acid sequence selected from the group consisting of CKIHAREIFDSRGNPTVEC (SEQ ID NO:4), CIHAREIFDSRGNPTVEC (SEQ ID NO:5) and CKIIGREILDSRGNPTVEC (SEQ ID NO:6) that is cyclised by a disulfide bound between the two cysteine residues.

2. The non citrullinated α-enolase polypeptide for use according to claim 1 which consists of the amino acid sequence SEQ ID NO:1 or the amino acid SEQ ID NO:2 or a function conservative variant thereof having at least 60% amino acid identity with SEQ ID NO:1 or SEQ ID NO:2.

3. The non citrullinated α-enolase polypeptide for use according to claim 1 or 2 wherein said autoimmune disease is rheumatoid arthritis.

## Patentansprüche

1. Ein nicht-citrulliniertes α-enolase Polypeptid zur Verwendung in einem Verfahren zur prophylaktischen Behandlung einer Autoimmunerkrankung in einem Subjekt mit Bedarf dafür, wobei das nicht-citrullinierte α-enolase Polypeptid umfasst:
eine Aminosäuresequenz, ausgewählt aus der Gruppe, bestehend aus der Aminosäuresequenz, die sich von Position 5 bis Position 21 in SEQ ID Nr:1 erstreckt, aus der Aminosäuresequenz, die sich von Position 6 bis Position 21 in SEQ ID Nr:2 erstreckt, und der Aminosäuresequenz, die sich von Position 5 bis Position 21 in SEQ ID Nr:3 erstreckt, oder
eine Aminosäuresequenz, ausgewählt aus der Gruppe, bestehend aus CKIHAREIFDSRGNPTVEC (SEQ ID Nr:4), CIHAREIFDSRGNPTVEC (SEQ ID Nr:5) und CKIIGREILDSRGNPTVEC (SEQ ID Nr:6), die zyklisiert ist durch eine Disulfidbrücke zwischen den zwei Cysteinresten.

2. Das nicht-citrullinierte α-enolase Polypeptid zur Verwendung nach Anspruch 1, welches besteht aus der Aminosäuresequenz SEQ ID Nr:1 oder der Aminosäuresequenz SEQ ID Nr:2 oder einer funktionskonsemativen Variante davon, die mindestens 60% Aminosäure-Sequenzidentität mit SEQ ID Nr: 1 oder SEQ ID Nr:2 hat.

3. Das nicht-citrullinierte α-enolase Polypeptid zur Verwendung nach Anspruch 1 oder 2, wobei die Autoimmunerkrankung rheumatoide Arthritis ist.

## Revendications

1. Polypeptide non citrulliné de l'α-énolase pour une utilisation dans un procédé de traitement prophylactique d'une maladie auto-immune chez un sujet en ayant besoin, ledit polypeptide non citrulliné de l'α-énolase comprenant :
une séquence d'acides aminés choisie dans le groupe constitué de la séquence d'acides aminés située dans la plage de la position 5 à la position 21 dans SEQ ID NO : 1, la séquence d'acides aminés située dans la plage de la position 6 à la position 21 dans SEQ ID NO : 2 et la séquence d'acides aminés située dans la plage de la position 5 à la position 21 dans SEQ ID NO : 3 ; ou
une séquence d'acides aminés choisie dans le groupe constitué de CKIHAREIFDSRGNPTVEC (SEQ ID NO : 4), CIHAREIFDSRGNPTVEC (SEQ ID NO : 5) ou CKIIGREILDSRGNPTVEC (SEQ ID NO : 6) qui est cyclisée par une liaison disulfure entre les deux résidus de cystéine.

2. Polypeptide non citrulliné de l'α-énolase pour une utilisation selon la revendication 1 qui est constitué de la séquence d'acides aminés SEQ ID NO : 1 ou de la séquence d'acides aminés SEQ ID NO : 2 ou de l'un de leurs variants fonctionnellement conservatifs présentant au moins 60 % d'identité des acides aminés avec SEQ ID NO : 1 ou SEQ ID NO : 2.

3. Polypeptide non citrulliné de l'α-énolase pour une utilisation selon la revendication 1 ou 2, où ladite maladie auto-immune est la polyarthrite rhumatoïde.


### SEQUENCE LISTING

<110> INSERM
<120> METHODS AND PHARMACEUTICAL COMPOSITIONS FOR THE TREATMENT OF AUTOIMMUNE DISEASES
<130> BI011173 VITTECOQ / MC
<160> 6
<170> PatentIn version 3.3
<210> 1
   <211> 434
   <212> PRT
   <213> Homo sapiens
<400> 1
   Met Ser Ile Leu Lys Ile His Ala Arg Glu Ile Phe Asp Ser Arg Gly
   1               5                   10                  15
   Asn Pro Thr Val Glu Val Asp Leu Phe Thr Ser Lys Gly Leu Phe Arg
               20                  25                  30
   Ala Ala Val Pro Ser Gly Ala Ser Thr Gly Ile Tyr Glu Ala Leu Glu
           35                  40                  45
   Leu Arg Asp Asn Asp Lys Thr Arg Tyr Met Gly Lys Gly Val Ser Lys
       50                  55                  60
   Ala Val Glu His Ile Asn Lys Thr Ile Ala Pro Ala Leu Val Ser Lys
   65                  70                  75                  80
   Lys Leu Asn Val Thr Glu Gln Glu Lys Ile Asp Lys Leu Met Ile Glu
                   85                  90                  95
   Met Asp Gly Thr Glu Asn Lys Ser Lys Phe Gly Ala Asn Ala Ile Leu
               100                 105                 110
   Gly Val Ser Leu Ala Val Cys Lys Ala Gly Ala Val Glu Lys Gly Val
           115                 120                 125
   Pro Leu Tyr Arg His Ile Ala Asp Leu Ala Gly Asn Ser Glu Val Ile
       130                 135                 140
   Leu Pro Val Pro Ala Phe Asn Val Ile Asn Gly Gly Ser His Ala Gly
   145                 150                 155                 160
   Asn Lys Leu Ala Met Gln Glu Phe Met Ile Leu Pro Val Gly Ala Ala
                   165                 170                 175
   Asn Phe Arg Glu Ala Met Arg Ile Gly Ala Glu Val Tyr His Asn Leu
               180                 185                 190
   Lys Asn Val Ile Lys Glu Lys Tyr Gly Lys Asp Ala Thr Asn Val Gly
           195                 200                 205
   Asp Glu Gly Gly Phe Ala Pro Asn Ile Leu Glu Asn Lys Glu Gly Leu
       210                 215                 220
   Glu Leu Leu Lys Thr Ala Ile Gly Lys Ala Gly Tyr Thr Asp Lys Val
   225                 230                 235                 240
   Val Ile Gly Met Asp Val Ala Ala Ser Glu Phe Phe Arg Ser Gly Lys
                   245                 250                 255
   Tyr Asp Leu Asp Phe Lys Ser Pro Asp Asp Pro Ser Arg Tyr Ile Ser
               260                 265                 270
   Pro Asp Gln Leu Ala Asp Leu Tyr Lys Ser Phe Ile Lys Asp Tyr Pro
           275                 280                 285
   Val Val Ser Ile Glu Asp Pro Phe Asp Gln Asp Asp Trp Gly Ala Trp
       290                 295                 300
   Gln Lys Phe Thr Ala Ser Ala Gly Ile Gln Val Val Gly Asp Asp Leu
   305                 310                 315                 320
   Thr Val Thr Asn Pro Lys Arg Ile Ala Lys Ala Val Asn Glu Lys Ser
                   325                 330                 335
   Cys Asn Cys Leu Leu Leu Lys Val Asn Gln Ile Gly Ser Val Thr Glu
               340                 345                 350
   Ser Leu Gln Ala Cys Lys Leu Ala Gln Ala Asn Gly Trp Gly Val Met
           355                 360                 365
   Val Ser His Arg Ser Gly Glu Thr Glu Asp Thr Phe Ile Ala Asp Leu
       370                 375                 380
   Val Val Gly Leu Cys Thr Gly Gln Ile Lys Thr Gly Ala Pro Cys Arg
   385                 390                 395                 400
   Ser Glu Arg Leu Ala Lys Tyr Asn Gln Leu Leu Arg Ile Glu Glu Glu
                   405                 410                 415
   Leu Gly Ser Lys Ala Lys Phe Ala Gly Arg Asn Phe Arg Asn Pro Leu
               420                 425                 430
   Ala Lys
<210> 2
   <211> 434
   <212> PRT
   <213> Mus musculus
<400> 2
   Met Ser Ile Leu Arg Ile His Ala Arg Glu Ile Phe Asp Ser Arg Gly
   1               5                   10                  15
   Asn Pro Thr Val Glu Val Asp Leu Tyr Thr Ala Lys Gly Leu Phe Arg
               20                  25                  30
   Ala Ala Val Pro Ser Gly Ala Ser Thr Gly Ile Tyr Glu Ala Leu Glu
           35                  40                  45
   Leu Arg Asp Asn Asp Lys Thr Arg Phe Met Gly Lys Gly Val Ser Gln
       50                  55                  60
   Ala Val Glu His Ile Asn Lys Thr Ile Ala Pro Ala Leu Val Ser Lys
   65                  70                  75                  80
   Lys Val Asn Val Val Glu Gln Glu Lys Ile Asp Lys Leu Met Ile Glu
                   85                  90                  95
   Met Asp Gly Thr Glu Asn Lys Ser Lys Phe Gly Ala Asn Ala Ile Leu
               100                 105                 110
   Gly Val Ser Leu Ala Val Cys Lys Ala Gly Ala Val Glu Lys Gly Val
           115                 120                 125
   Pro Leu Tyr Arg His Ile Ala Asp Leu Ala Gly Asn Pro Glu Val Ile
       130                 135                 140
   Leu Pro Val Pro Ala Phe Asn Val Ile Asn Gly Gly Ser His Ala Gly
   145                 150                 155                 160
   Asn Lys Leu Ala Met Gln Glu Phe Met Ile Leu Pro Val Gly Ala Ser
                   165                 170                 175
   Ser Phe Arg Glu Ala Met Arg Ile Gly Ala Glu Val Tyr His Asn Leu
               180                 185                 190
   Lys Asn Val Ile Lys Glu Lys Tyr Gly Lys Asp Ala Thr Asn Val Gly
           195                 200                 205
   Asp Glu Gly Gly Phe Ala Pro Asn Ile Leu Glu Asn Lys Glu Ala Leu
       210                 215                 220
   Glu Leu Leu Lys Thr Ala Ile Ala Lys Ala Gly Tyr Thr Asp Gln Val
   225                 230                 235                 240
   Val Ile Gly Met Asp Val Ala Ala Ser Glu Phe Tyr Arg Ser Gly Lys
                   245                 250                 255
   Tyr Asp Leu Asp Phe Lys Ser Pro Asp Asp Pro Ser Arg Tyr Ile Thr
               260                 265                 270
   Pro Asp Gln Leu Ala Asp Leu Tyr Lys Ser Phe Val Gln Asn Tyr Pro
           275                 280                 285
   Val Val Ser Ile Glu Asp Pro Phe Asp Gln Asp Asp Trp Gly Ala Trp
       290                 295                 300
   Gln Lys Phe Thr Ala Ser Ala Gly Ile Gln Val Val Gly Asp Asp Leu
   305                 310                 315                 320
   Thr Val Thr Asn Pro Lys Arg Ile Ala Lys Ala Ala Ser Glu Lys Ser
                   325                 330                 335
   Cys Asn Cys Leu Leu Leu Lys Val Asn Gln Ile Gly Ser Val Thr Glu
               340                 345                 350
   Ser Leu Gln Ala Cys Lys Leu Ala Gln Ser Asn Gly Trp Gly Val Met
           355                 360                 365
   Val Ser His Arg Ser Gly Glu Thr Glu Asp Thr Phe Ile Ala Asp Leu
       370                 375                 380
   Val Val Gly Leu Cys Thr Gly Gln Ile Lys Thr Gly Ala Pro Cys Arg
   385                 390                 395                 400
   Ser Glu Arg Leu Ala Lys Tyr Asn Gln Ile Leu Arg Ile Glu Glu Glu
                   405                 410                 415
   Leu Gly Ser Lys Ala Lys Phe Ala Gly Arg Ser Phe Arg Asn Pro Leu
               420                 425                 430
   Ala Lys
<210> 3
   <211> 425
   <212> PRT
   <213> Porphyromonas gingivalis
<400> 3
   Met Glu Ile Ala Lys Ile Ile Gly Arg Glu Ile Leu Asp Ser Arg Gly
   1               5                   10                  15
   Asn Pro Thr Val Glu Val Asp Val His Leu Ala Cys Gly Ile Ile Gly
               20                  25                  30
   Arg Ala Ala Val Pro Ser Gly Ala Ser Thr Gly Glu Asn Glu Ala Ile
           35                  40                  45
   Glu Leu Arg Asp Gln Asp Lys Ala Arg Tyr Cys Gly Lys Gly Val Leu
       50                  55                  60
   Lys Ala Val Lys Asn Val Asn Glu Val Ile Asp Pro Ala Leu Cys Gly
   65                  70                  75                  80
   Met Ser Val Leu Glu Gln Thr Ala Ile Asp Arg Lys Leu Ile Glu Leu
                   85                  90                  95
   Asp Gly Thr Lys Thr Lys Ser Asn Leu Gly Ala Asn Ala Met Leu Gly
               100                 105                 110
   Val Ser Leu Ala Val Ala Lys Ala Ala Ala Ala Tyr Leu Asp Ile Pro
           115                 120                 125
   Leu Tyr Arg Tyr Ile Gly Gly Ser Asn Thr Tyr Val Leu Pro Val Pro
       130                 135                 140
   Met Met Asn Ile Ile Asn Gly Gly Ser His Ser Asp Ala Pro Ile Ala
   145                 150                 155                 160
   Phe Gln Glu Phe Met Ile Arg Pro Val Gly Ala Cys Cys Phe Arg Glu
                   165                 170                 175
   Gly Leu Arg Met Gly Ala Glu Val Phe His Ala Leu Lys Lys Val Leu
               180                 185                 190
   His Asp Arg Gly Leu Ser Thr Ala Val Gly Asp Glu Gly Gly Phe Ala
           195                 200                 205
   Pro Ala Leu Asn Gly Thr Glu Asp Ala Ile Glu Ser Ile Leu Lys Ala
       210                 215                 220
   Val Glu Ala Ala Gly Tyr Val Pro Gly Lys Asp Ile Thr Ile Ala Met
   225                 230                 235                 240
   Asp Cys Ala Ser Ser Glu Phe Phe Lys Asp Gly Ile Tyr Asp Tyr Thr
                   245                 250                 255
   Lys Phe Glu Gly Glu Lys Gly Lys Lys Arg Ser Ile Asp Glu Gln Val
               260                 265                 270
   Ala Tyr Leu Thr Glu Leu Val Gly Lys Tyr Pro Ile Asp Ser Ile Glu
           275                 280                 285
   Asp Gly Met Ser Glu Asn Asp Trp Glu Gly Trp Lys Lys Leu Thr Val
       290                 295                 300
   Ala Leu Gly Asp Lys Val Gln Leu Val Gly Asp Asp Leu Phe Val Thr
   305                 310                 315                 320
   Asn Val Glu Phe Leu Arg Arg Gly Ile Ala Glu Lys Cys Gly Asn Ser
                   325                 330                 335
   Ile Leu Ile Lys Val Asn Gln Ile Gly Thr Leu Thr Glu Thr Leu Asn
               340                 345                 350
   Ala Ile Glu Met Ala His Arg His Gly Phe Thr Ser Val Thr Ser His
           355                 360                 365
   Arg Ser Gly Glu Thr Glu Asp Thr Thr Ile Ala Asp Ile Ala Val Ala
       370                 375                 380
   Thr Asn Ser Gly Gln Ile Lys Thr Gly Ser Leu Ser Arg Thr Asp Arg
   385                 390                 395                 400
   Met Ala Lys Tyr Asn Gln Leu Leu Arg Ile Glu Glu Glu Leu Gly Pro
                   405                 410                 415
   Cys Ala Val Tyr Gly Tyr Lys Lys Val
               420                 425
<210> 4
   <211> 19
   <212> PRT
   <213> Artificial
<220>
   <223> chEP1
<400> 4
   Cys Lys Ile His Ala Arg Glu Ile Phe Asp Ser Arg Gly Asn Pro Thr
   1 5 10 15
   Val Glu Cys
<210> 5
   <211> 18
   <212> PRT
   <213> Artificial
<220>
   <223> cmEP1
<400> 5
   Cys Ile His Ala Arg Glu Ile Phe Asp Ser Arg Gly Asn Pro Thr Val
   1 5 10 15
   Glu Cys
<210> 6
   <211> 19
   <212> PRT
   <213> Artificial
<220>
   <223> cPorEP1
<400> 6
   Cys Lys Ile Ile Gly Arg Glu Ile Leu Asp Ser Arg Gly Asn Pro Thr
   1 5 10 15
   Val Glu Cys